# EUROPEAN PATENT SPECIFICATION

(11) **EP 2 234 479 B1**
(45) Date of publication and mention of the grant of the patent: **16.03.2016**
(21) Application number: 08864047.9
(22) Date of filing: 22.12.2008
(51) Int. Cl.: A01N 35/02, A01N 37/06, A01N 65/00, C07H 15/256, A61K 36/25

(54) **COMPOSITION AND METHOD**
ZUSAMMENSETZUNG UND METHODE
COMPOSITION ET PROCÉDÉ

(30) Priority: 21.12.2007 GB 0724967
(43) Date of publication of application: 06.10.2010
(73) Proprietor: Bangor University, Bangor Gwynedd LL57 2UW (GB)
(72) Inventor: BAIRD, Mark, Stephen, Bangor, Gwynedd LL57 2EZ (GB); PRESKETT, David, Bangor, Gwynedd LL57 4BP (GB)
(74) Representative: Appleyard Lees
(86) International application number: PCT/GB2008/051224
(87) International publication number: WO 2009/081211

(56) References cited:
- WO-A-01/60153
- AT-B- 401 456
- GB-A- 2 051 575
- US-A- 5 290 557
- TAKECHI ET AL: "Structure-activity relationships of the saponin alpha-hederin" PHYTOCHEMISTRY, PERGAMON PRESS, GB, vol. 29, no. 2, 1 January 1990 (1990-01-01), pages 451-452, XP022515587 ISSN: 0031-9422
- TIMON-DAVID P ET AL: "RECHERCHE D'UNE ACTIVITE ANTIFONGIQUE DE PLUSIEURS PRINCIPES ACTIFS EXTRAITS DU LIERRE GRIMPANT: HEDERA HELIX L.//RESEARCH OF ANTIFUNGAL ACTIVITY FROM SEVERAL ACTIVE PRINCIPLE EXTRACTS FROM CLIMBING-IVY: HEDERA HELIX L" ANNALES PHARMACEUTIQUES FRANCAISES, MASSON, PARIS, FR, vol. 38, no. 6, 1 January 1980 (1980-01-01), pages 545-552, XP009041929 ISSN: 0003-4509
- ZEHAVI U. ET AL: "Synthesis and antifungal activity of medicagenic acid saponins on plant pathogens: Modification of the saccharide moiety and the 23[alpha] substitution" CARBOHYDRATE RESEARCH, vol. 244, 1992, pages 161-169, XP002528924
- BEDIR E ET AL: "Triterpene saponins from the fruits of Hedera helix" PHYTOCHEMISTRY, PERGAMON PRESS, GB, vol. 53, no. 8, 1 April 2000 (2000-04-01), pages 905-909, XP004291398 ISSN: 0031-9422
- SUNIL RATNAYAKE ET AL: "Petroselinic Acid from the Ripe Berries of Aralia spinosa" PHARMACEUTICAL BIOLOGY, SWETS AND ZEITLINGER, LISSE, NL, vol. 31, no. 1, 1 January 1993 (1993-01-01), pages 35-37, XP009116922 ISSN: 1388-0209
- SONG S-J ET AL: "Five saponins from the root bark of Aralia elata" PHYTOCHEMISTRY, PERGAMON PRESS, GB, vol. 56, no. 5, 1 March 2001 (2001-03-01), pages 491-497, XP004291794 ISSN: 0031-9422
- CRESPIN F ET AL: "HIGH-PERFORMANCE LIQUID CHROMATOGRAPHIC DETERMINATION OF SAPONINS FROM HEDERA HELIX L. USING A LIGHT-SCATTERING DETECTOR" CHROMATOGRAPHIA, WIESBADEN, DE, vol. 38, no. 3/04, 1 February 1994 (1994-02-01), pages 183-186, XP009041930 ISSN: 0009-5893
- ELIAS R ET AL: "INFLUENCE DU PROCEDE DE SECHAGE ET DU DEGRE ALCOOLIQUE SUR L'EXTRACTION DE L'HEDERASAPONINE C ET DE L'ALPHA-HEDERINE A PARTIR DES FEUILLES DE HEDERA" JOURNAL DE PHARMACIE DE BELGIQUE, vol. 46, no. 3, 1 January 1991 (1991-01-01), pages 177-181, XP009033524 ISSN: 0047-2166
- HOSTETTMANN K.: "Saponins with molluscicidal activity from Hedera helix L." HELVETICA CHIMICA ACTA, vol. 63, no. 3, 1980, pages 606-609, XP002528925
- GÖKÇE A; WHALON M E; ÇAM H; YANAR Y; DEMIRTAS I; GÖREN N: "Plant extract contact toxicities to various developmental stages of Colorado potato beetles (Coleoptera: Chrysomelidae)" ANNALS OF APPLIED BIOLOGY, vol. 149, 2006, pages 197-202, XP002528926
- VILLANI M; GOULD F: "SCREENING OF CRUDE PLANT EXTRACTS AS FEEDING DETERRENTS OF THE WIREWORM MELANOTUS-COMMUNIS" ENTOMOLOGIA EXPERIMENTALIS ET APPLICATA, vol. 37, 1985, pages 69-75, XP002528927
- EVANGELIA RÖHNER ET AL: 'Effectiveness of plant extracts of Paeonia suffruticosa and Hedera helix against diseases caused by Phytophthora infestans in tomato and Pseudoperonospora cubensis in cucumber' JOURNAL OF PLANT DISEASES AND PROTECTION vol. 111, no. 1, 01 January 2004, pages 83 - 95, XP055031610 ISSN: 0340-8159
- R. Elias ET AL: "Triterpenoid Saponins from the Leaves of Hedera helix", Journal of Natural Products, vol. 54, no. 1, 1 January 1991 (1991-01-01), pages 98-103, XP055196243, ISSN: 0163-3864, DOI: 10.1021/np50073a006

## Description

The present invention relates to compounds obtainable from the *Araliaceae* family of plants, to compositions comprising the same and to uses therefor.

The *Araliaceae* family of plants comprises two subfamilies, the *Araliodideae* and the *Hydrocotyloideae* subfamilies. The genera of plants covered by the *Araliodideae* subfamily include *Anakasia, Apiopetalum, Aralia, Arthrophyllum, Astrotricha, Boninofatsia, Brassaiopsis, Cephalaralia, Cheirodendron, Cromapanax, Cuphocarpus, Cussonia, Dendropanax, Eleutherococcus,* × *Fatshedera, Fatsia, Gamblea, Gastonia, Harmsiopanax, Hedera, Heteropanax, Hunaniopanax, Kalopanax, Mackinlaya, Macropanax, Megalopanax, Merrilliopanax, Meryta, Metapanax, Motherwellia, Munroidendron, Oplopanax, Oreopanax, Osmoxylon, Panax, Polyscias, Pseudopanax, Pseudosciadium, Raukaua, Reynoldsia, Schefflera, Sciadodendron, Seemannaralia, Sinopanax, Stilbocarpa, Tetrapanax, Tetraplasandra, Trevesia* and *Woodburnia.*

The subfamily *Hydrocotyloideae* includes the genera *Azorella, Centella, Hydrocotyle, Platysace* and *Xanthosia.*

Of the genera of species in the *Araliaceae* plant family, the present invention relates in particular to those of the *Hedera* genus. Species of the *Hedera* genus include *Hedera algeriensis, Hedera azorica, Hedera canariensis, Hedera caucasigena, Hedera colchica, Hedera cypria, Hedera helix, Hedera hibernica, Hedera maderensis, Hedera maroccana, Hedera nepalensis, Hedera pastuchowii, Hedera rhombea, Hedera sinensis* and *Hedera taurica.*

A wide variety of plant extracts are commonly used in numerous medicinal and industrial applications.

One class of useful compounds obtainable from a number of plants are saponins.

Saponins are so named due to their ability to form stable, soap-like foams at low concentrations (Latin sapo = soap); this ability was used as a quantitative assay of saponins and forms the basis of much literature on the subject.

The nomenclature of saponins was reviewed by Hostettmann and Marston in Saponins, p 10-17, in Phillipson, J.D. (ed.) Chemistry and Pharmacology of Natural Products (series), CUP, Cambridge, 1995, starting with two basic skeletons; steroidal, having 27 carbon atoms or triterpenoid, with 30 carbon atoms. The less widely distributed steroidal forms are further divided into two classes, the spirostanes, found principally in monocotyledons such as lilies, onions, yucca and agave and the furostanes. This latter steroidal group are of considerable commercial importance as a platform molecule for the manufacture of steroid hormones, principally from *Dioscorea.* A recent review has classified dammaranes, lupanes, hopanes, oleananes, ursanes and steroids according to their biosynthetic pathways.

The triterpenes are a larger group consisting of two principal structural classes; either tetracyclic or pentacyclic. Tetracyclic structures are sub-divided as dammaranes, cucurbitanes and lanostanes. The pentacyclic structure forms the largest single group, the oleananes. Other major pentacyclic triterpene classes are ursanes and lupanes while minor classes of taraxeranes, taraxastanes and friedolanes are also recognised.

The core skeleton molecules are termed aglycones (also referred to as genins or sapogenins) and are not usually found without substituents attached. The carbon skeleton of the most common aglycones of the oleanane class are shown in figure 1.

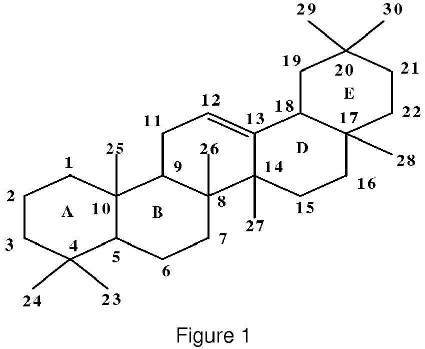

Figure 1 shows the carbon skeleton of the olean-12-en aglycone structure. Principal attachments are most commonly found at the C3 and C28 positions. Other attachment points are usually linked to an OH group, typically at the C2, C23 or C24 positions.

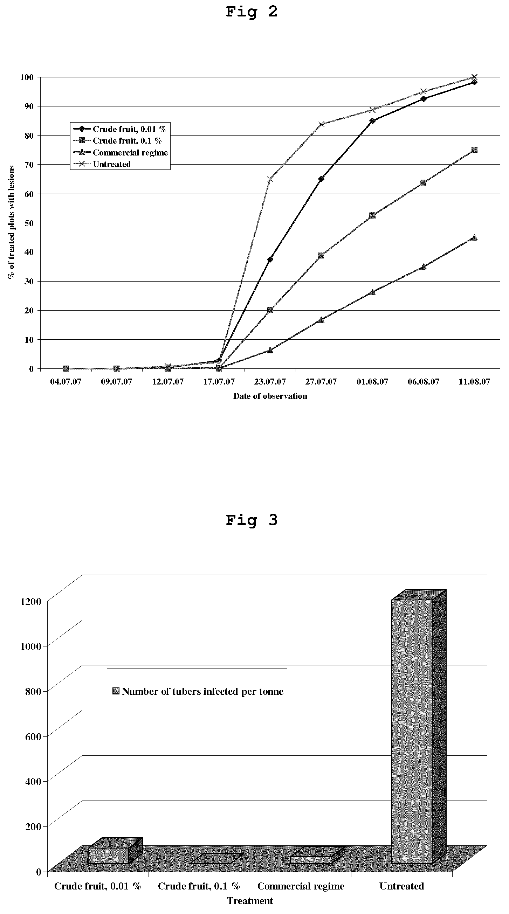

Of the oleananes, which occur in most orders of the plant kingdom, the aglycones most commonly found are oleanolic acid shown in figure 2, followed by hederagenin shown in figure 3.

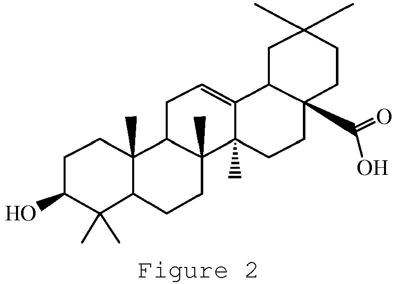

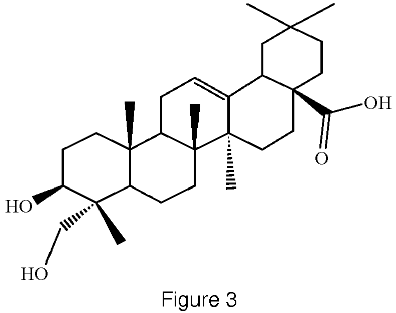

In broad terms, sugars are the principal substituents found generally either as monosaccharides or as polysaccharide chains, although in *Calendula officianalis* (marigolds), saturated fatty acids are sometimes bound to the aglycone at the C3 position. It will be appreciated however that natural sources of saponins comprise complex mixtures of compounds and that the amounts of different compounds present in a sample will vary considerably from species to species. They also vary within the different parts of the plant.

The present invention relates to the extraction, modification and use of saponin compounds from plants of the *Araliaceae* family, *Hedera helix.*

The antifungal effects of the saponin-rich extracts from the leaves of *H. helix* are well reported, for example, by P. Timon-David et al in Annales pharmaceutiques françaises, 1980, 38 (6), 545-552. Such extracts were found to be active against *phytophthora infestans* by E. Röhner et al in the Journal of Plant Diseases and Protection, 2004, 111 (1), 83-95. K. Hostettmann reported molluscicidal activity of extracts from the fruits of *H. helix* in Helvetica Chimica Acta, 1980, 63 III (60), 606-609. E. Bedir reported that the fruit extracts contain triterpene saponins and investigated their control of *Candida albicans* in Phytochemistry, 2000, 53, 905-909. However, control of late potato blight by the saponin-rich extracts of the fruits has not been reported.

According to a first aspect of the present invention, there is provided a method of combating late potato blight comprising the steps of
(i) obtaining a saponin-rich component from *Hedra helix* by a process comprising the steps of:
   (a) treating a portion of the fruit of the plant with an extraction solvent in which saponin-containing compounds are soluble; and
   (b) treating the portion of plant or the extract obtained therefrom to remove fatty acid residues from said portion of plant or extract thereof; and
(ii) applying to a crop a composition comprising a saponin rich component obtained by the process of step (i).

The portion of plant comprises fruits of the plant. It may comprise the whole fruit, including the fleshy pulp and the seed or it may comprise only the fleshy pulp part of the fruit.

In some embodiments the portion of plant consists essentially of fruit, which may be whole fruit including seeds or only the fleshy portion of the fruit.

The portion of plant may be harvested by any suitable means. It may be harvested by hand or by mechanical means, for example using flails, combined harvesting, by beating or by cutting. Vacuum assisted methods could also be used.

Examples of suitable extraction solvents for use in step (a) include pyridine, THF, DMSO and alcohols.

Preferably the extraction solvent comprises an alcohol. More preferably the extraction solvent comprises an alcohol having 1 to 4 carbon atoms. Most preferably the extraction solvent comprises methanol or ethanol. The extraction solvent may comprise neat methanol and/or ethanol or it may comprise an aqueous solution thereof. For example it may comprise at least 80%, more preferably at least 95% alcohol. In some embodiments the extraction solvent comprises at least 95 wt% ethanol. For example, absolute ethanol or 99 wt% ethanol may be used. In other embodiments, an aqueous alcohol may be used, for example comprising from 50 to 90%, preferably 65 to 85% ethanol by volume.

Preferably the portion of plant is formed into a comminuted form prior to step (a). This may involve taking a sample of the plant and forming it into a paste, for example using a food processor, a pestle and mortar or mincer. Alternatively the plant may be chopped or shredded using a knife or other cutting implement. In some preferred embodiments the plant is processed by hammermilling or grinding into the comminuted form.

In preferred embodiments the portion of plant is dried prior to step (a). This may be before and/or after the plant is formed into a comminuted form. Preferably the portion of plant is processed to provide a comminuted form after drying.

Such a drying step may comprise heating the portion of plant in an oven. Typically this may be for at least an hour, preferably at least four hours, more preferably at least ten hours, for example at least sixteen hours, preferably at least twenty hours. Drying may comprise heating in an oven for up to a week, for example up to three days, for example up to forty hours, for example up to thirty hours.

The drying step may involve heating in an oven at a temperature of at least 35°C, preferably at least 40°C, for example at least 50°C. The drying step may be carried out in an oven having a temperature of up to 250°C, preferably up to 200°C, for example up to 150°C, or up to 120°C. Oven temperatures of 50-60°C or 80-90°C may typically be used. Preferably air is circulated over the portion of plant during the drying process.

In some embodiments step (a) may be carried out at ambient temperature.

Preferably however step (a) comprises heating a portion of the plant in the extraction solvent. This may be at a temperature of at least 30ºC, preferably at least 35 ºC, more preferably at least 40°C, for example at least 50°C, preferably at least 60°C. The extraction may be carried out by heating at a temperature of up to 150°C, for example up to 120°C, for example up to 100°C, for example up to 90°C, or up to 80°C. Suitably step (a) comprises heating a portion of plant in a refluxing solvent.

The extraction step (a) is suitably carried out by heating a portion of plant in the extraction solvent for at least 1 hour, for example at least 6 hours, preferably at least 10 hours, more preferably at least 18 hours, for example at least 30 hours.

The plant extract may be heated in the solvent for up to a week, for example up to 5 days, preferably up to 3 days.

Step (a) may comprise heating a portion of the plant in an extraction solvent for more than one period. A further solvent sample may be added and the heating repeated.

Preferably step (a) involves continuous extraction of the saponin compounds. Preferably it is carried out using apparatus which allows percolation of the solvent and soaking of the portion of plant therein. The portion of plant may be suspended loosely in the solvent or held within a removable container.

In some embodiments step (a) may not comprise simply heating the portion of plant in an extraction solvent. If using a supercritical solvent, for example supercritical carbon dioxide, heating may not be necessary. The use of supercritical carbon dioxide as a reaction solvent has a number of advantages, for example it is non-toxic, can be allowed to simply evaporate at the end of a reaction and may allow reactions to be carried out at lower temperatures.

Step (a) may include the use of a microwave or a sonicator with or without heating to assist extraction of saponin-containing compounds into the extraction solvent.

A review paper, Recent advances in extraction of nutraceuticals from plants, Lijun Wang and Curtis L. Weller, Trends in Food Science & Technology, 17 (2006), 300-312, details a number of extraction methods which could suitably be used in step (a) of the process of the present invention.

Suitably the mass of plant heated in the solvent in step (a) is at least 50 g/L, for example at least 80 g/L, preferably at least 100 g/L. Mass ratios of up to 2000 g/L, for example up to 1000 g/L or 500 g/L are suitable. Mass ratios of for example 100 g/L to 400 g/L may be used.

Step (b) comprises treating the portion of plant or an extract obtained therefrom to remove fatty acid residues from said portion of plant or extract thereof.

By fatty acid residues we mean to refer to compounds comprising fatty acids, that is long chain (for example greater than 4 carbon atoms) aliphatic moieties including an acid functionality. The fatty acid residues may be present as the free acid, salts or esters thereof, including monoesters, diesters and triesters. Phospholipids may also be present. Most commonly fatty acid residues are present as glycerol triesters.

Steps (a) and (b) may be carried out in any order. In some embodiments they may be carried out simultaneously. Preferably they are carried out sequentially. Preferably step (b) is carried out after step (a). Preferably step (b) comprises treating the extract obtained in step (a) to remove fatty acid residues.

In embodiments in which step (a) is carried out first, the extract obtained in step (a) suitably comprises saponin-containing compounds, fatty acid residues and the extraction solvent. The extraction solvent may be removed, for example under reduced pressure to provide a concentrated extract comprising saponin-containing compounds and fatty acid residues. This concentrated extract may further comprise other constituents, for example one or more of free sugars, acetylenic compounds, proteins, flavanoids, chlorophyll and lignocellulosic compounds. However these other constituents are suitably present in minor amounts, for example less than 25 wt%, preferably less than 10 wt%, more preferably less than 5 wt%. In a preferred embodiment in which the extraction solvent used comprises at least 98 wt% ethanol, and the portion of plant comprises the fruits of *Hedera Helix,* the concentrated extract comprises from 20 to 60 wt%, for example 35 to 45 wt% saponin-containing compounds and from 40 to 80 wt%, for example 55 to 65 wt% fatty acid residues.

The process is carried out on the fruits of *Hedera Helix.* The weight ratio of saponin-containing compounds to fatty acid residues in the extract obtained in step (a) is suitably from 5:1 to 1:5, preferably from 2:1 to 1:2. This material may itself be of commercial utility as a source of saponin-containing compounds, and could be used, where appropriate in any of the applications described herein.

In some embodiments in which step (a) comprises heating the portion of plant in the extraction solvent, when the extract obtained in step (a) is allowed to cool, the fatty acid residues become less soluble and come out of solution. A separate layer may be observed forming in the extract, typically a lower layer which suitably comprises glycerol triesters of fatty acid compounds. These materials may be soluble in hot solvent, for example ethanol but become less soluble as the extract cools. Thus step (b) may comprise leaving the extract to cool. Preferably the extract is allowed to cool slowly. Once an oily layer has formed, for example at the bottom of the extract, this can be readily separated leaving a saponin-rich component in the settling vessel. Alternatively the saponin-rich component may be decanted off.

In some cases the volume of solvent in the extract obtained in step (a) may be reduced, for example by at least 25% or at least 50%, prior to carrying out step (b).

In an alternative embodiment step (b) may comprise concentrating the extract obtained in step (a) either partially or substantially to dryness and then washing the residue with a solvent having a lower polarity than the extraction solvent. Suitable solvents include hexane or other hydrocarbons, mixtures of hydrocarbons (for example those commonly known as petroleum ether), diethyl ether, ethyl acetate, and halogenated solvents (for example dichloromethane or chloroform) and acetone. Suitably the extract obtained in step (a) is first concentrated by removal of the solvent *in vacuo.*

In some embodiments of the first aspect of the present invention, step (b) may be carried out before step (a). In such embodiments the portion of plant is suitably treated to remove fatty acid residues and then the same portion of plant is subjected to step (a).

In embodiments in which step (b) is carried out first, a portion of plant, which is preferably in comminuted form and dried as described above, is suitably treated with a solvent in which fatty acid residues have a higher solubility than do saponin-containing compounds. Preferably fatty acid residues are substantially soluble in said solvent and saponin-containing compounds are substantially insoluble. Preferred solvents are hexane and mixtures of hydrocarbons, especially mixtures of alkanes, for example those having a boiling point of 40-80ºC. Suitably in such embodiments, the portion of plant is heated in the solvent, for example at reflux, typically for a period of 1 to 24, for example 2 to 4 hours.

In some embodiments a portion of plant is heated in a series of solvents of increasing polarity incorporating as such steps (a) and (b). For example, the portion of plant may be heated first in hexane, then dichloromethane, followed by ethyl acetate and then ethanol. The ethanol fraction would be expected to be rich in saponin-containing compounds, with fatty acid residues having been extracted using the previous solvents.

In some embodiments the process of the present invention further comprises repeating steps (a) and/or step (b).

In embodiments of the process of the first aspect of the present invention in which step (b) comprises removing fatty acid residues from the extract obtained in step (a), preferably at least 50 wt% of fatty acid residues originally present in the extract are removed, preferably at least 70 wt%, more preferably at least 80 wt%, preferably at least 90 wt% and most preferably at least 95 wt%.

Suitably in step (b), along with removal of fatty acid residues, there is concurrent removal of other non-saponin species.

An advantage of the process of the present invention is that the fatty acid residues removed in step (b) are themselves of considerable commercial utility. Thus the removal of fatty acid residues in step (b) could be regarded as a separation of saponin-containing compounds and fatty acid residues.

Thus in some preferred embodiments, the process provides a method of obtaining a saponin-rich component and a fatty acid-rich component from a plant of the *Araliaceae* family using a single extraction procedure.

The crude fatty acid component obtained in step (b) may include fatty acid residues of petroselinic acid, vaccenic acid and palmitoleic acid. If the portion of plant consists essentially of whole ripe fruit of *Hedera Helix,* it would be expected that these three acids would each be present in an amount of 20 to 40 wt% as the free acid or an ester thereof, especially a glycerol triester of one or more of these acids. This crude fatty acid component may find utility as a biofuel for example biodiesel, a dietary additive, a nutraceutical, a cosmetic base, a lubricant, or feedstock for industrial processes, for example the manufacture of surfactants. The ozonolysis products of these materials may also be of commercial utility. For example ozonolysis of petroselinic acid provides adipic acid, a precursor to nylon; and lauric acid which is used to make the surfactant sodium lauryl sulphate. Fatty acid esters obtained in step (b) could also be interesterified with other triglyceride stocks to formulate specialised mixtures having applications in food manufacture.

Some members of the *Araliaceae* plant family have been found to contain high concentrations of compounds of petroselinic acid. The fruits and in particular the seeds of *Hedera helix* have been found to include high concentrations of esters of petroselinic acid. Levels are highest when ripe or mature fruit are used.

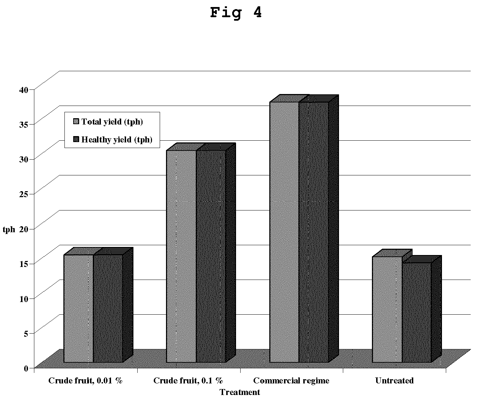

Petroselinic acid has the formula shown in figure 4.

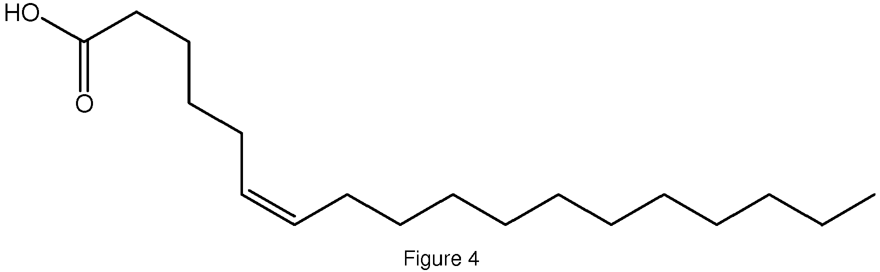

Petroselinic acid is a useful material. It is monounsatured but has similar physical characteristics to saturated fatty acids at room temperature. Petroselinic acid and derivatives thereof, especially glycerol triesters, may be used to replace saturated fats in, for example, dietary applications. It may also be used as a substitute for partially hydrogenated fats. Partially hydrogenated fats often include a double bond having a trans configuration. These are known to be damaging to human health if ingested on a regular basis.

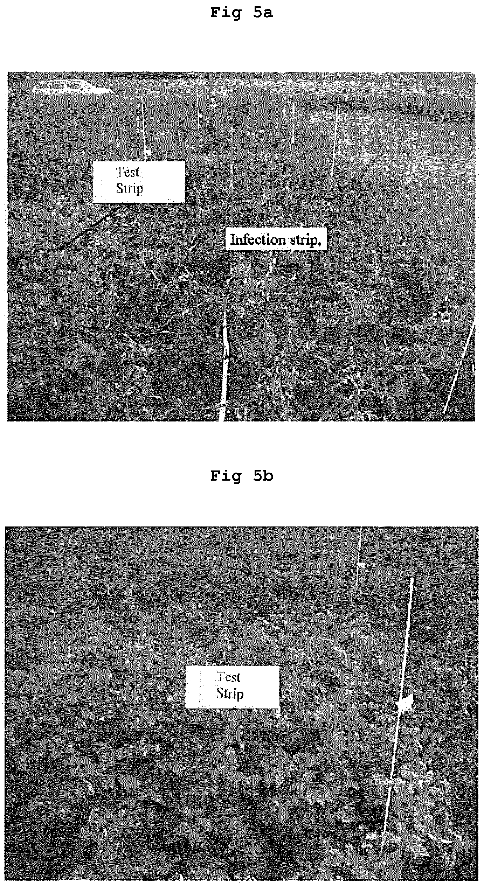

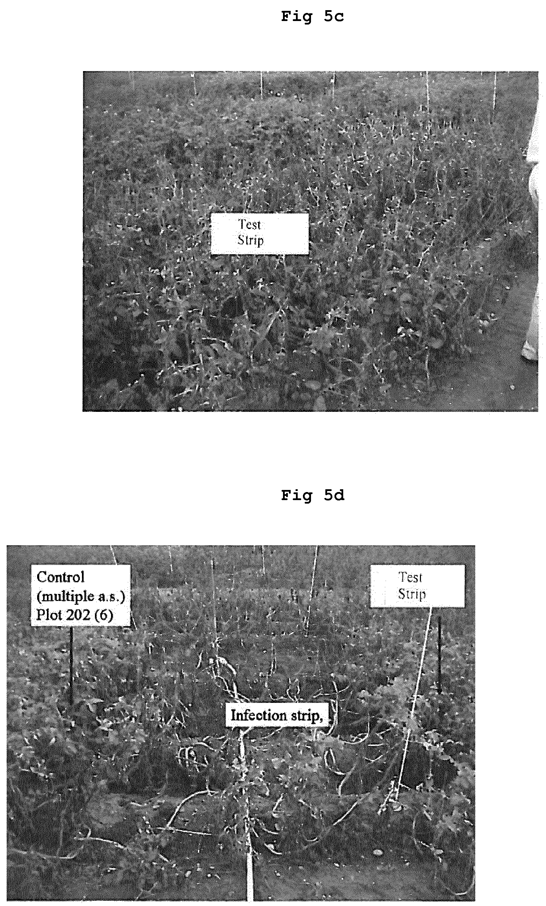

The present inventors have found that species of the *Araliaceae* family, in particular the fruits, and especially the seeds of *Hedera helix,* include high concentrations of the glycerol triester of petroselinic acid, known as tripetroselinin, that is the compound having the formula shown in figure 5. Indeed the present inventors have found that the seeds of *Hedera helix* may contain up to 80 wt% of triglycerides comprising petroselinic acid.

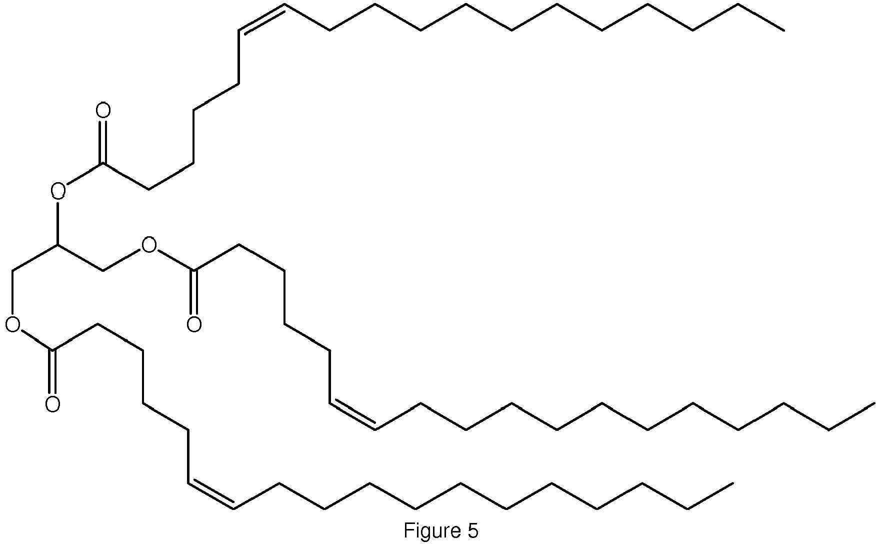

Previous methods of obtaining this compound from natural sources involved extracting the petroselinic acid as a free acid (after hydrolysis), along with other fatty acids; followed by a complex separation of petroselinic acid from the other fatty acids; and then esterifying to the glycerol triester. In another method of the prior art, tripetroselinin was recovered by molecular distillation, although the yield was poor. Petroselinic acid has been obtained from fennel seeds by acid soap crystallisation in methanol, followed by two urea segregations. The present inventors have found that using the process of the present invention, it is possible to obtain the glycerol triester of petroselinic acid in crystalline form without the need for hydrolysis and esterification, molecular distillation, interesterification, or the use of a co-crystallisation agent such as urea.

A component rich in fatty acid residues is obtained in step (b) of the process and is useful as a crude mixture. The crude mixture may contain a number of compounds including principally the glycerol triesters of petroselinic acid, vaccenic acid and palmitoleic acid, as well as mixed glycerol triesters of two or three of these acids. In some embodiments the crude mixture may be purified by methods known to those skilled in the art to provide the constituent fatty acids and/or esters thereof. In particular the process is useful for providing the glycerol triester of petroselinic acid, the glycerol triester of vaccenic acid, the glycerol triester of palmitoleic acid, and mixed triesters.

The glycerol triesters thus obtained could be further reacted. For example they could be hydrolysed under acidic or basic conditions to give the free acid. This free acid could then be further reacted, for example to form a monoester. Methyl esters of fatty acids are useful as biodiesel. Monoesters could alternatively be obtained by transesterification of the glycerol triesters. Such subsequent reactions could be carried out directly on the component obtained in step (b) or on the constituent triesters after separation thereof. In some cases, subsequent reaction of the mixture may assist separation. It is possible that the component obtained in step (b) comprises mixed esters in which two or more different acids are bound to a single glycerol molecule.

In particular, when step (b) is carried out on seeds from *Hedera helix* or an extract thereof, the resultant fatty acid residue has been found to contain high levels of glycerol esters of petroselinic acid.

The present inventors have found that the triglycerol ester of petroselinic acid may be isolated from the fatty acid containing residue obtained in step (b) as a crystalline solid. This can be achieved by dissolving the fatty acid residue in a crystallisation solvent with or without heating, and then cooling the resultant solution to a temperature of less than 5ºC, preferably less than 0°C, suitably preferably less than -5ºC, for example -10ºC or -20ºC. A crystalline product forms which can be collected by filtration, decanting or centrifuge and recrystallised if necessary to increase the purity thereof. The filtrate from the initial crystallisation may be rich in other fatty acid derivatives, including compounds containing residues of vaccenic and palmitoleic acids, for example the glycerol triester of vaccenic acid and the glycerol triester of palmitoleic acid. Other triglyceride components may be isolated separately from the residue obtained in step (b).

Any suitable solvent may be used as the crystallisation solvent. Suitable solvents include ketones, for example acetone; alcohols, for example ethanol; ethers, for example tetrahydrofuran or diethyl ether; esters, for example ethyl acetate; cholorinated solvents, for example dichloromethane; and alkanes, for example hexane or heptane and mixtures of alkanes. A preferred crystallisation solvent is acetone.

Preferably the glycerol triester of petroselinic acid formed by this method is at least 60% pure, preferably at least 70%, more preferably at least 90% and most preferably at least 95% pure, for example at least 99% pure.

A similar method may be used to obtain petroselinic acid as the free acid in high purity. The crude fatty acid residue obtained in step (b) may be hydrolysed under acidic or basic conditions to provide a mixture of free fatty acids. This mixture may be dissolved in a crystallisation solvent, with or without heating and cooled to a temperature of less than 5 ºC, for example less than 0 ºC or less than -5 ºC. Preferred crystallisation solvents are as described above. The resultant precipitate, which may be collected by filtration, will be rich in petroselinic acid, and can be recrystallised to further increase the purity. It is thus possible to obtain petroselinic acid having a purity in excess of 70%, for example in excess of 90% or 95%.

The present invention uses a saponin-rich component.

Preferably the saponin-rich component comprises less than 10 wt% fatty acid residues.

Preferably the component comprises less than 8 wt% fatty acid residues, more preferably less than 5 wt%, preferably less than 3 wt%, for example less than 2 wt%, preferably less than 1.5 wt%, preferably less than 1 wt% and most preferably 0.5 wt%.

Suitably the saponin-rich component comprises at least 20 wt% saponin compounds, preferably at least 30 wt%, more preferably at least 40 wt%, preferably at least 50 wt%, preferably at least 60 wt%, more preferably at least 70 wt% and most preferably at least 80 wt%. By saponin compounds, we mean to refer to all compounds which include a central saponogenic core, for example the aglycone core of figure 1. The molecules may be further substituted with pendant side groups. The component of the second aspect preferably comprises compounds which have the structures shown in figures 2 and 3 and those which include sugar residues bound to one or more of the hydroxy moieties. Compounds present include monodesmosides in which a single sugar is pendant, for example at the C3 position, and bidesmosides in which two sugar residues are found, for example, at the C3 and C28 positions. These pendant sugar residues may be monosaccharides, disaccharides or polysaccharides and may typically include one or more of arabinose, rhamnose, galactose, glucose, xylose, mannose and fructose. The relative proportion of monodesmosides and bidesmosides and the nature of the attached sugars depends on which part of the plant is used.

Preferably the saponin-rich component comprises at least 40 wt% saponin-containing compounds having a triterpene structure, preferably at least 50 wt%, more preferably at least 60 wt%, for example at least 70 wt% or at least 80 wt%.

Preferably the component comprises less than 20 wt% of steroidal terpenes, preferably less than 10 wt%, more preferably less than 5 wt%, preferably less than 2.5 wt%, preferably less than 1 wt% and most preferably less 0.5 wt% steroidal terpenes.

The composition is preferably an aqueous composition.

In some embodiments the composition may be prepared by dissolving the saponin-rich in a small volume of water-miscible solvent (for example an alcohol) in which it is readily soluble and then diluting with water to form a substantially aqueous composition.

The composition may comprise from 0.00001 to 5 wt% of saponin-containing compounds obtained from the fruit of *Hedera Helix.*

The invention may involve the use of a concentrated formulation which upon dilution forms a composition comprising a saponin-rich component. This may be provided in the form of a solid or liquid, for example a powder, a suspension, a solution or an emulsion.

The composition may suitably be varied according to the intended use thereof. It may further comprise one or more components selected from a water miscible solvent (for example ethanol, propanol or polyethylene glycol), an antioxidant (for example tocopherol or BHT), a chelating agent (for example EDTA), a dispersant, a surfactant and an emulsifier.

In some embodiments the composition may further comprise an adhesion-promoting agent. This may be included, for example in an aqueous composition which is applied to a crop.

Suitable adhesion-promoting agents are materials which enable the composition to stick to a substrate more readily. The substrate may for example be a plant and an adhesion-promoting agent will help prevent the composition from running off the plant and/or being washed away in rainfall. Suitable compounds for use as adhesion-promoting agents include natural rubber latex or synthetic latex.

The adhesion-promoting agent is preferably present in the composition in an amount of from 0.001 to 5 wt%, for example 0.005 to 2 wt%, preferably 0.01 to 0.2 wt%.

A polymeric material may have bound thereto or within saponin-containing compounds obtained from fruits of *Hedera Helix.* The saponin-containing compounds may, for example be covalently bound to a polymeric residue. In such embodiments the polymer may be formed into a plastic plant mat or band which could be attached to a plant that is desired to be protected against molluscs.

Alternatively, the polymer may comprise a latex-like material having dispersed therein saponin-containing compounds. This may be applied to ligno-cellulosic materials, for example wood fibre, or other material, for example agricultural wastes formed into fibre as mats to bind the ligno-cellulosic materials. These may be laid on or around the plants. The plants may also be planted directly through the mats.

The present inventors have discovered that the saponin-rich component shows significant advantages in combating late potato blight.

The present invention may provide the use of a composition comprising a saponin-rich component in combating late potato blight.

By combating late potato blight we mean to include preventing the occurrence of, inhibiting the growth of and controlling late potato blight. Late potato blight is also known as *Phytophthora infestans.*

The present invention provides a method of combating late potato blight, the method comprising applying to a crop a composition comprising a saponin-rich component.

Suitably the method comprises applying a composition comprising from 0.0001 to 5 wt%, for example 0.0005 to 1 wt%, preferably 0.001 to 0.05 wt%, more preferably 0.001 to 0.25 wt% of the saponin-containing component to the crop.

Suitably in the method of combating potato blight of the present invention the composition is applied to the crop once every 3 to 12 days, for example once every 5 to 9 days, for example every 6 to 8 days, for example every 7 days.

Typically a crop will be treated for a period of at least 4 weeks, preferably a period of at least 6 weeks, for example for a period 8 to 16 or 10 to 12 weeks.

The method of combating potato blight of the present invention may include treating the crop on some occasions with a composition comprising a saponin-rich component and on other occasions with a composition comprising a different agent able to combat potato blight. In some embodiments a composition comprising a saponin-rich component and a composition comprising another agent able to combat potato blight may be coapplied. Suitably in the method of the present invention, between 1 in 2 and 1 in 6 treatments of a crop may include applying a composition comprising a saponin-rich component.

In the method of combating potato blight of the present invention, the composition may be applied to the crop by any suitable means known to those skilled in the art. One suitable method is spraying the crop. Typically each hectare of crop will be sprayed with between 100 and 1000 g, preferably between 200 and 500 g, for example about 300 g of the component of the second aspect or the hydrolysis product of the third aspect. This would be applied in dilute form as a composition of the fourth aspect. Typically a dried powder composition is diluted with water and then applied to the crop, for example at a concentration of 0.1 to 10 g/L.

The crude extract obtained in step (a) of the process of the first aspect could be incorporated into a composition for combating blight.

The present invention will now be further described by way of the following non limiting examples.

### Example 1 - Extraction of dried, prepared H. helix fruits

Ripe fruits (15.00 kg, 69.34 % moisture content) were collected from various locations on Anglesey in April 2005 and dried in a 50 °C oven for two days, reducing the moisture content (m.c.) to 4.43 %. They were then minced to a meal (4.59 kg) in a food processor and a portion of the meal (94.16 g) taken for immediate extraction. The remainder was placed in an airtight container and frozen for future use.

On defrosting, two samples of prepared meal (30.00 g and 60.00 g) were extracted separately and exhaustively in a Soxhlet apparatus for 48 hours using EtOH (99%, 250 ml) at reflux. Following removal of the solvent *in vacuo,* the pasty solid recovered was washed in petrol (50 ml X 3) and filtered through a Buchner funnel under reduced pressure. The solid recovered was dried in an oven at 50 °C overnight then recovery of crude saponin component recorded. The solvent wash was removed *in vacuo* and the recovery of crude fatty acid residue recorded. Table 1 shows the amount of each component recovered.

**Table 1**

| **Sample** | **Crude Saponin (g)** | **Recovery saponin (%) at o.d.w.** | **Crude fatty acid (g)** | **Recovery fatty acid (%) at o.d.w.** |
|---|---|---|---|---|
| 30 g | 7.60 | 26.51 | 7.69 | 26.82 |
| 60 g | 16.93 | 29.53 | 17.62 | 30.72 |

The presence of saponins was confirmed by ¹H-NMR spectroscopy showing typical signals of this class of compound against standards of hederagenin, α-hederin and hederacoside C.

Transesterification of the fatty acid component to the respective fatty acid butyl esters and subsequent GCMS analysis against fatty acid methyl ester standards and comparison with literature data for butyl esters showed principally petroselinic acid (30 g = 28.73 %, 60 g = 29.37 %), palmitoleic acid (30 g = 20.43 %, 60 g = 20.16 %) and vaccenic acid (30 g = 16.99 %, 60 g = 16.84 %).

By contrast, under the same conditions for recoveries of crude saponins and fatty acid components from fruits respectively, a methanol extract afforded 29.89 % and 11.27 % but from 2-propanol 28.73 % and 33.98 % were recovered.

### Example 2

On a larger scale, fruits (26.45 kg, 67.8 % m.c.), were prepared as described in relation to example 1 above to obtain dried fruits that were frozen until used. The meal was thawed and a portion (6.58 g) taken for m.c. determination, establishing 7.44 %. With stirring (Heidolph RZR2102 overhead stirrer at 91 r.p.m.) the remaining dried material (7.87 kg) was charged to a 50 L jacketed vessel (Diehm, Wertheim), holding 35 L of EtOH. The vessel was heated using silicone oil and a heater unit (Huber Wright 141) to 50 °C and left, with stirring for six hours. It was then switched off and left to cool overnight. The vessel was then drained down and the solvent removed on a rotary evaporator stepwise to obtain a thick paste. 26.97 L of EtOH were recovered. The paste was washed exhaustively using 40-60 petrol. The solution was filtered through a Buchner funnel under reduced pressure and the solvent removed on a rotary evaporator to obtain a green oil (537.92 g, 6.84 % o.d.w.). The remaining paste was removed from the flask to an evaporating basin then placed in a 50 °C oven for three days. After grinding in a pestle and mortar, a fine, amorphous, purple powder (1,176.20 g, 14.94 %) was recovered. A ¹H-NMR spectrum of this confirmed the product was principally saponins.

The recovered solvent was returned to the vessel and the extraction repeated at 50 °C without stirring as the solids had compacted. The solvent sat above the filter cake and percolated through slowly. It was drained down over two days and treated as above to recover EtOH (21.06 L), a green oil (322.83 g, 4.10 % o.d.w.) and a brown solid (384.05 g, 4.88 % o.d.w.). ¹H-NMR of the solid confirmed the saponins were present as the principal component. The recovery of fatty acid residues can be improved by draining of the vessel at higher temperatures. Crude saponin recovery at nearly 20 % is less than that obtained at Soxhlet conditions though had the solvent been drawn down hot, recovery of both components would have been improved.

### Example 5 - Control of P. infestans (late blight) with a crude fruit extract.

Dried ivy fruit meal (94g) was extracted with EtOH (99%, 3 x 250 mL) with stirring. The extract was washed with petrol (x3), dried and concentrated to provide 21 g of a solid material which appeared to be predominantly saponin-containing compounds by ¹H and ¹³C NMR spectroscopy.

The activity of the crude, defatted fruit extract against *Phytophthora infestans* was studied using a detached leaf assay. Crude extract (1.25 g) was dissolved in EtOH (10 ml) with gentle heating then made up with de-ionised water to 62.50 ml. A dilution series from the 2 % solution was prepared of 0.200 %, 0.020 % and 0.002 % concentrations. Leaves were removed from the stems of *Solanum tuberosum* var. Bintje, a Dutch potato susceptible to blight. The leaves were washed then air-dried following which they were treated with the concentration of the extract to the point of run-off then dried. The leaves were placed with the lower surface uppermost in a tray lined with moistened tissue. An aqueous suspension of sporangia (1 ml) having a concentration of 20 sporangia/µl was applied to the centre of each leaf and the tray covered. Three leaves per treatment were used and a control of ethanol (10 ml) made up to 62.5 ml with de-ionised water was included. The trial ran for twelve days by which stage the leaves had become necrotic with secondary infections.

The results after eight days following inoculation showed clearly that the control leaves were infected with lesions of *P. infestans.* The lowest concentration (0.002 %) had one leaf infected with lesions, two others were uninfected. In all other cases, no infection of the leaves was observed. The trial continued up to twelve days showing no further infection.

### Example 6

In an independent field trial the crude fruit extract was applied at concentrations (w/v) of 0.1 % and 0.01 % in water (3 L) plus an adhesion agent, (sold under the trade mark Bond) at 0.14 % for each treatment concentration. Plots (four replicates of 0.08 hectares each per treatment) were planted at density of 5 plants per linear metre x 4 rows per plot on 09.05.07.

Two control plots were included in the trial: an untreated control and a control treated with a commercial regime of different, proprietary fungicides applied thus to prevent development of resistance - the details of the commercial regime are shown in table 3.

**Table 3**

| **Application** | | |
|---|---|---|
| **Treatment** | **rate** | **Quantity** |
| Shirlan | 0.4 | l/ ha. |
| Curzate M | 2 | kg/ ha. |
| Invader | 2 | kg/ ha. |
| Ranman A | 0.2 | l/ha. |

| | | |
|---|---|---|
| Shirlan (RTM) is a suspension concentrate containing 500 g/l (38.4% w/w) fluazinam. Curzate M (RTM) is a mixture of Mancozeb (68 %), Cymoxanil (4.5 %) and Hexamethylenetetramine (3.4 %). Invader (RTM) contains 75 g / kg dimethomorph and 667 g / kg mancozeb. Ranman A (RTM) is a suspension concentrate (SC) containing 400 g/l Cyazofamid. | | |

Treatments were applied at seven day intervals from 27.06.07 until 16.07.07 then decreasing to five day intervals during the highest blight pressure of July and August until 07.08.07 (*n* = 9 applications). The application rate of the extracts was 300 g/hectare and 30 g/hectare respectively; the sprayer volume rate was 300 L/hectare. The crop was inoculated on 06.07.07 with a suspension of *P. infestans* sporangia sprayed onto 2 m wide infection strips running between the trial plots. The first infection in the untreated plots was found on 12.07.07. Following the final treatment, when conditions were no longer conducive to further infestation, the crops were left to develop, sprayed with a desiccant and finally lifted for tuber assessment on 11.10.07.

The development of leaf lesions by *P. infestans* was strongly inhibited by the commercial regime; control (no treatment) plots were completely destroyed by the infection. The crude fruit extract applied at 0.1 % showed comparable activity to the commercial regime up to the middle of July then continued to provide significant protection until the end of treatments. This was not so with the extract at a 0.01 % rate although some activity was observed relative to the untreated control. Figure 2 shows lesion development of *P. infestans* on trial plots (n = 4 plots per treatment).

Examination of the data that followed desiccation of the haulms and harvesting of the tubers shows that the commercial regime and the 0.01 % crude extract were largely successful in preventing tuber blight; in the case of the 0.1 % extract, this gave total protection. Extrapolation of the data shown in figure 3 showed that the number of tubers infected by blight per tonne was zero in the case of the 0.1% crude extract and outperformed even the control regime. Even at 0.01 %, very high beneficial efficacy was recorded relative to the untreated plots.

The number of tubers expressed per tonne per hectare (tph) shows in figure 4 that the 0.1 % crude extract has activity comparable to that of the control regime. When crude fruit extract is present at 0.01%, there is little infection of the tubers.

These results show clearly that there is a very significant effect by the crude extract formulations and they exhibit a strong dose response between them. Their action may have been partly due to the adhesion agent; however, this was used in both formulations at 0.14 %. It was also used (at 0.1 %) on a further 16 plots that were run concurrently with these trials examining other, unrelated formulations. In all cases, plots were decimated by *P. infestans* and no dose response was seen between them. It was also noted, that natural *P. infestans,* in addition to the inoculum, was high throughout the trial period at the site; rainfall was reported for the site as 462.6 mm against a thirty year average of 179.9 mm. The 2007 season was one of the wettest in many years and as such the combination of the prevalence of *P. infestans'* sporangia, high humidity, temperature and constant, inclement weather were ideal conditions for infestation over the trial period and meant failure for many potato growers.

Figures 5a to d shows pictures of the plots towards the end of the trial.

Fig 5a shows a plot I treated with a composition comprising 0.1 wt% of a saponin component obtained according to Example 2 and an adjacent infection strip;

Fig 5b shows plot I more closely - it can clearly be seen that the plants are in a condition similar to those treated with a commercial regime;

Fig 5c shows a plot II treated with a composition comprising 0.1 wt% of a saponin component obtained according to Example 2; and

Fig 5d shows plot I, an adjacent infection strip and a control plot treated with a currently used commercial formulation for combating late potato blight.

## Claims

1. A method of combating late potato blight comprising the steps of
(i) obtaining a saponin-rich component from *Hedra* helix by a process comprising the steps of:
(a) treating a portion of the fruit of the plant with an extraction solvent in which saponin-containing compounds are soluble; and
(b) treating the portion of plant or the extract obtained therefrom to remove fatty acid residues from said portion of plant or extract thereof; and
(ii) applying to a crop a composition comprising a saponin rich component obtained by the process of step (i).

2. A method according to claim 1 wherein the extraction solvent comprises an alcohol.

3. A method according to claim 1 or claim 2 in which step (a) is carried out prior to step (b).

4. A method of combating late potato blight, accordingly to any preceding claim, the method comprising applying to a crop a composition comprising from 0.0005 to 1 wt% of a saponin rich component obtained by the process of step (i).

5. A method according to any preceding claim wherein the composition is applied to the crop once every 3 to 12 days.

6. The use of a saponin rich component obtained by step (i) of claim 1 in combating late potato blight.

## Patentansprüche

1. Verfahren zum Bekämpfen der Kraut- und Knollenfäule der Kartoffel, umfassend die folgenden Schritte:
(i) Gewinnen einer saponinreichen Komponente aus *Hedra* helix mittels eines Verfahrens, das die folgenden Schritte umfasst:
(a) Behandeln eines Teils der Frucht der Pflanze mit einem Extraktionslösungsmittel, in dem saponinhaltige Verbindungen löslich sind; und
(b) Behandeln des Teils der Pflanze oder des hiervon erhaltenen Extrakts, um Fettsäurereste von dem Teil der Pflanze oder dem Extrakt davon zu entfernen; und
(ii) Aufbringen einer Zusammensetzung, die eine saponinreiche Komponente, die nach dem Verfahren von Schritt (i) erhalten wurde, umfasst, auf eine Pflanzenkultur.

2. Verfahren nach Anspruch 1, wobei das Extraktionslösungsmittel einen Alkohol umfasst.

3. Verfahren nach Anspruch 1 oder Anspruch 2, in dem Schritt (a) vor Schritt (b) erfolgt.

4. Verfahren zum Bekämpfen der Kraut- und Knollenfäule der Kartoffel nach einem vorhergehenden Anspruch, wobei das Verfahren umfasst, dass man eine Zusammensetzung, die 0,0005 bis 1 Gew.-% einer saponinreichen Komponente, die nach dem Verfahren von Schritt (i) erhalten wurde, umfasst, auf eine Pflanzenkultur ausbringt.

5. Verfahren nach einem vorhergehenden Anspruch, wobei die Zusammensetzung einmal alle 3 bis 12 Tage auf die Pflanzenkultur ausgebracht wird.

6. Verwendung einer saponinreichen Komponente, die nach Schritt (i) von Anspruch 1 erhalten wurde, zur Bekämpfung der Kraut- und Knollenfäule der Kartoffel.

## Revendications

1. Procédé de lutte contre le mildiou de la pomme de terre comprenant les étapes de
(i) obtention d'un composant riche en saponine de *Hedra helix* par un processus comprenant les étapes de :
(a) traitement d'une partie du fruit de la plante avec un solvant d'extraction dans lequel les composés contenant de la saponine sont solubles ; et
(b) traitement de la partie de plante ou de l'extrait obtenu à partir de celle-ci pour éliminer les résidus d'acide gras de ladite partie de plante ou extrait de celle-ci ; et
(ii) application sur une culture d'une composition comprenant un composant riche en saponine obtenu par le processus de l'étape (i).

2. Procédé selon la revendication 1 dans lequel le solvant d'extraction comprend un alcool.

3. Procédé selon la revendication 1 ou la revendication 2 dans lequel l'étape (a) est conduite avant l'étape (b).

4. Procédé de lutte contre le mildiou de la pomme de terre, selon l'une quelconque des revendications précédentes, le procédé comprenant l'application sur une culture d'une composition comprenant de 0,0005 à 1 % en poids d'un composant riche en saponine obtenu par le processus de l'étape (i).

5. Procédé selon l'une quelconque des revendications précédentes dans lequel la composition est appliquée sur la culture une fois tous les 3 à 12 jours.

6. Utilisation d'un composant riche en saponine obtenu par l'étape (i) de la revendication 1 dans la lutte contre le mildiou de la pomme de terre.
